# EUROPEAN PATENT SPECIFICATION

(11) **EP 3 044 192 B1**
(45) Date of publication and mention of the grant of the patent: **18.10.2017**
(21) Application number: 14799192.1
(22) Date of filing: 10.09.2014
(51) Int. Cl.: C07B 59/00

(54) **DEUTERATED COMPOUNDS**
DEUTERIERTE VERBINDUNGEN
COMPOSÉS DEUTÉRÉS

(30) Priority: 10.09.2013 GB 201316050
(43) Date of publication of application: 20.07.2016
(73) Proprietor: Swansea University, Swansea SA2 8PP (GB)
(72) Inventor: GRIFFITHS, William, Swansea SA2 8PP (GB); WANG, Yuqin, Swansea SA2 8PP (GB); THEOFILOPOULOS, Spyridon, Swansea SA2 8PP (GB); NORMANTON, John, Swansea SA2 8PP (GB); ARENAS, Ernest, Swansea SA2 8PP (GB)
(74) Representative: CSY London
(86) International application number: PCT/GB2014/000356
(87) International publication number: WO 2015/036726

(56) References cited:
- SHODA J ET AL: "Synthesis of potential C27-intermediates in bile acid biosynthesis and their deuterium-labeled analogs", STEROIDS, ELSEVIER SCIENCE PUBLISHERS, NEW YORK, NY, US, vol. 58, no. 3, 1 March 1993 (1993-03-01), pages 119-125, XP027269937, ISSN: 0039-128X [retrieved on 1993-03-01]

## Description

### Field of the Invention

The present invention relates to deuterated compounds and in particular compounds that are derivatives of cholestenoic acids. The present invention also relates to compositions including said deuterated compounds and said compounds for use, in particular but not exclusively, in the treatment of conditions such as motor neurone disease.

### Background of the Invention

The vertebrate central nervous system is composed of a wide variety of neurons that are generated following tightly-regulated developmental programs. One means of developmental and adult regulation is via nuclear receptors. Nuclear receptors expressed in embryonic and adult brain have a developmental role and a function in the adult brain. The liverX receptors (Lxra and β), which are activated by oxysterols, are required for neurogenesis during ventral midbrain (VM) development. It is believed that Lxrs and their ligands have an effect on adult motor neurons. Enzymes involved in the synthesis of cholesterol and oxysterols, such as 2,3-oxidosqualene-lanosterol cyclase, are localized in Islet1+ oculomotor neurons in the mouse VM at E11.5 and oxysterols and endogenous brain Lxr ligands are sufficient to regulate neurogenesis in the developing ventral midbrain. While endogenous brain Lxr ligands have been identified and found to regulate the development of midbrain dopaminergic neurons and red nucleus neurons, no endogenous ligand capable of regulating the survival of motor neurons *in vivo* has so far been identified but research has indicated that specific Lxr ligands regulate the balance between motor neuron survival and death.

Cholestenoic acids are not mere intermediate metabolites of bile acid biosynthesis, but are rather a diverse family of bioactive compounds, capable of regulating nuclear receptor function. They were found to specifically activate Lxr and elicit an array of functions ranging from the regulation of Islet-1 expression, to the positive and negative regulation of motor neuron survival. It has been shown that 3β,7α-dihydroxycholest-5-en-26-oic acid (3β,7α-diHCA) and 3β,7β-dihydroxycholest-5-en-26-oic acid (3β,7β-diHCA) are Lxr ligands present in human CSF (cerebrospinal fluid). Of these, 3β,7β-diHCA, and the previously identified Lxr ligand, 3β-hydroxycholest-5-en-26-oic acid (3β-HCA), were found to cause neuronal cell death, and the latter was present at high levels in patients with hereditary spastic paresis type SPG5. Also 3β,7α-diHCA, which was found at low levels in SPG5 and was absent from cerebrotendinous xanthomatosis (CTX) patients was found to promote motor neuron survival. This indicates that compounds such as cholestenoic acids may be Lxr ligands and as such are key regulators of motor neuron function in development and disease. However, the neuroprotective 3β,7α-diHCA is metabolised by enzymes such as HSD3B7 to its 3-oxo metabolite 7α-hydroxy-3-oxocholest-4-en-26-oic acid (7αH,3O-CA) or to its neurotoxic metabolite 3β,7β-diHCA. Alternatively, metabolism may lead to side-chain shortening through beta oxidation to C₂₄ acids.

Many current medicines suffer from poor absorption, distribution, metabolism and/or excretion that prevent their wider use or limit their use in therapeutic composition. One strategy for improving such drug's metabolic properties is to stabilise the compounds and a particular stabilizer is the deuterium atom. As the size and shape of deuterium is essentially similar to that of hydrogen, replacement of hydrogen by deuterium would not be expected to have an effect on the biochemical potency and selectivity of the drug as compared with the original chemical entity that contains only hydrogen.

Steroids (1993) 58(3), 119-125 discloses the preparation of deuterated 27-hydroxycholesterol by Clemmensen reduction of kryptogenin using deuterium oxide, deuteroethanol and deuterium chloride.

The present invention seeks to overcome problems with the prior art by stabilising cholestenoic acids whilst also providing neuroprotective properties for those compounds.

### Summary of the Invention

According to a first aspect of the invention there is provided compound of general formula (I) 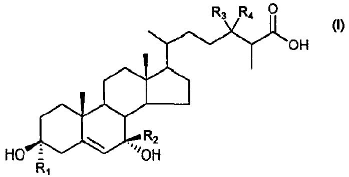 or a pharmaceutically acceptable salt thereof, wherein:
R1 - R4 are each independently selected from H and deuterium; and
at least one of R1 - R4 is deuterium.

It is envisaged that R1 is deuterium and R2-R4 is hydrogen.

As an alternative it is envisaged that R2 is deuterium and R1, R3, R4 is hydrogen.

In an alternative arrangement each of R1 andR2 are deuterium and R3 and R4 are hydrogen.

It is similarly envisaged that R3 and/or R4 is deuterium giving additional arrangement of isotopes.

Preferably the compound of formula (I) is a cholestenoic acid.

It is preferred that the compound of formula (I) is deuterated 3β,7α-dihydroxycholest-5-en-26-oic acid (3β,7α-diHCA) and pharmaceutically acceptable salts thereof.

It is envisaged that any atom not designated as deuterium is to be considered to be present at or close to its natural abundance.

Alternatively any hydrogen atom may optionally be substituted for a deuterium atom at an abundance that is greater than the natural abundance of deuterium.

The compounds are useful for treating both humans and other mammals for example farm animals or pets and so according to a second aspect of the invention there is provided a compound of formula (I) for use in medicine and in particular for the treatment of neurodegenerative diseases.

According to a third aspect there is provided the use of a compound of general formula (I) in the preparation of an agent for the treatment of neurodegenerative conditions.

Furthermore disclosed is a method for the treatment of neurodegenerative diseases, the method comprising administering to a patient in need of such treatment, an effective amount of a compound of general formula (I).

According to a fifth aspect of the invention there is provided a process for the preparation of a pharmaceutical composition comprising bringing a compound of general formula (I) in conjunction or association with a pharmaceutically or veterinarily acceptable carrier or vehicle.

To conclude, this invention relates to deuterated compounds, and in particular to forms of 3β,7α-dihydroxycholest-5-en-26-oic acid (3β,7α-diHCA) and pharmaceutically acceptable salts thereof. This invention also provides compositions comprising a compound of this invention and the use of such compositions in methods of treating diseases and conditions that are beneficially treated by administering a deuterated form of 3β,7α-dihydroxycholest-5-en-26-oic acid (3β,7α-diHCA) to treat illnesses such as motor neurone disease

The invention will now be described in greater detail with reference to the following examples and to drawings in which:

### Brief Description of the Drawings

The invention will now be described by way of example only with reference to and as illustrated in the following figures and examples in which;
**Figure 1** shows: a schematic pathway showing the breakdown of cholesterol in the body and the effect of compounds that are produced; and
**Figure 2** shows: specific cholestenoic acids increase the number of Islet1+ oculomotor neurons, promote neuronal survival, or are toxic in mouse E11.5 brain primary cultures.

### Detailed Description of an embodiment of the Invention

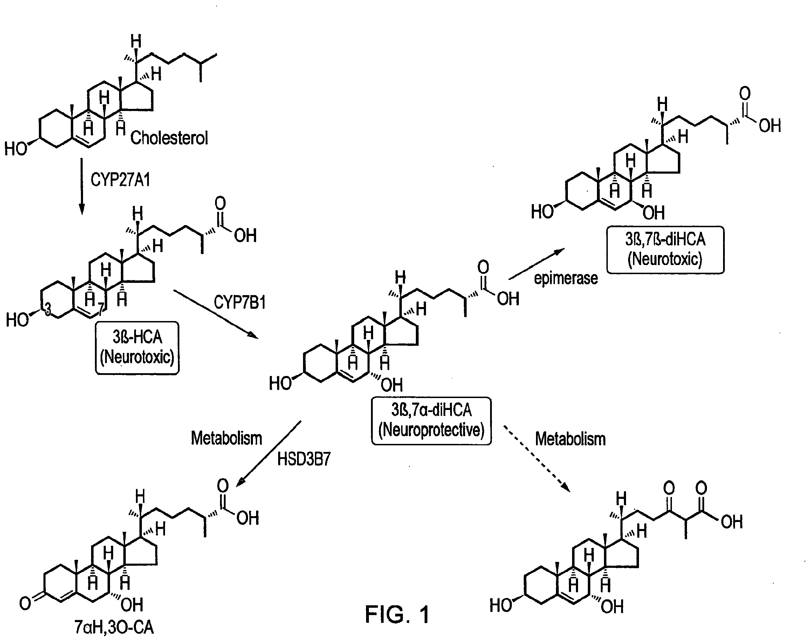

**Figure 1** shows a schematic pathway showing the breakdown of cholesterol in the body and the effect of compounds that are produced. As can be seen, both neurotoxic and neuroprotective compounds can be produced which may be in different ratios. The invention relates to deuterated compounds and in particular cholestenoic acids which have deuterated groups and the compounds that are deuterated are represented by those of formula (I). "Deuterated" (also referred to as D or ²H) means that at least one of the atoms in the compound is deuterium in an abundance that is greater than the natural abundance of deuterium (typically approximately 0.015%). "Deuterium" refers to an isotope of hydrogen wherein the nucleus contains one proton and one neutron.

The concentration of deuterium at a designated position may be defined by the deuterium enrichment factor. "Deuterium enrichment factor" in the context used herein refers to the ratio between the deuterium isotopic abundance and the natural abundance of deuterium, each relative to hydrogen abundance.

If a substituent in a compound of this invention is denoted as deuterium, such compound has a deuterium enrichment factor for each designated deuterium atom of:
at least 1000 (15% deuterium incorporation at each designated deuterium atom);
at least 2000 (30% deuterium incorporation at each designated deuterium atom);
at least 3000 (45% deuterium incorporation at each designated deuterium atom);
at least 4000 (60% deuterium incorporation at each designated deuterium atom);
at least 5000 (75% deuterium incorporation at each designated deuterium atom);
at least 6000 (90% deuterium incorporation at each designated deuterium atom);
at least 6466.7 (97% deuterium incorporation at each designated deuterium atom);
at least 6600 (99% deuterium incorporation at each designated deuterium atom); or
at least 6633.3 (99.5% deuterium incorporation at each designated deuterium atom).

As stated according to a first aspect of the invention the compounds are of general formula (I) but there may be following variations of the compound or a pharmaceutically acceptable salt thereof, where R1 - R4 are each independently selected from H and deuterium; and at least one of R1 - R4 is deuterium.

These compounds are shown as compound (II) to (IX):

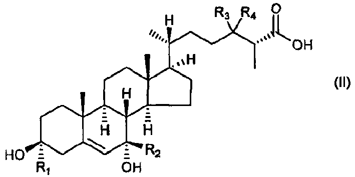

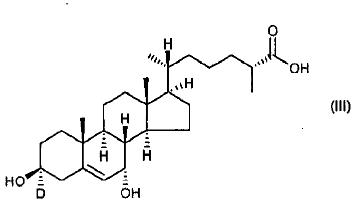

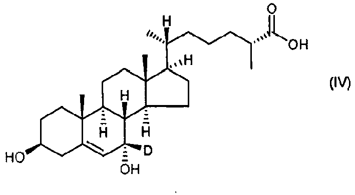

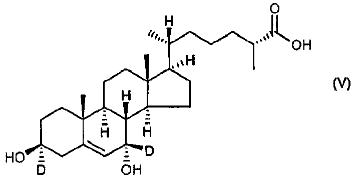

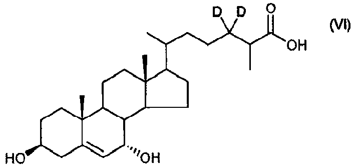

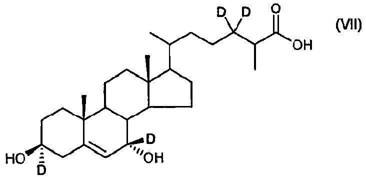

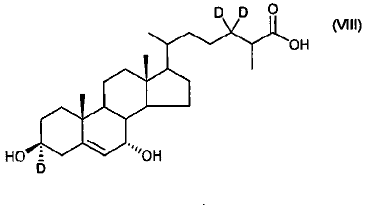

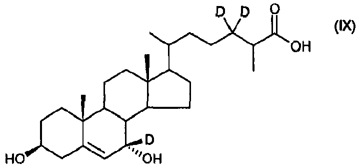

For the compound of the invention there may be isotopic variation among the constituent atoms of the molecules. Thus the extent of deuteration at each designated position is not necessarily the same. By way of example, when a compound of the present invention contains multiple deuterium atoms, the deuterium enrichment factor for one designated deuterium atom may be 3000 and the deuterium enrichment factor for another designated deuterium atom may be 5000. Such a compound is considered therefore to have a deuterium enrichment factor of 3000.

The structural formulae shown herein may or may not indicate whether atoms at certain positions are isotopically enriched with respect to deuterium. Thus in a general embodiment, when a structural formula is silent with respect to whether a particular position is isotopically enriched, it is to be understood that the stable isotopes at that particular position are present at, or close to, natural abundance or alternatively "D" indicates that the stable isotopes at that particular position are deuterium enriched.

The structural formulae shown herein may or may not indicate specific enantiomers for chiral centres where such chiral centres exist. Where a chiral centre exists but enantiomeric specificity is not designated it is understood that both or all chiral enantiomers are encompassed by the formula. As such, compounds of the present invention can exist as either individual enantiomers, or mixtures of the two enantiomers. Accordingly, a compound of the present invention may exist as either a racemic mixture or a scalemic mixture, or as individual respective stereoisomers that are substantially free from another possible stereoisomer. The term "substantially free of other stereoisomers" as used herein means less than 25% of other stereoisomers, preferably less than 10% of other stereoisomers, more preferably less than 5% of other stereoisomers and most preferably less than 2% of other stereoisomers are present. Methods of obtaining or synthesizing an individual enantiomer for a given compound are known in the art and may be applied as practicable to final compounds or to starting material or intermediates.

Compounds of the present invention may be formulated to treat or provide a therapy for a range of motor neurone diseases. "Treat" or "therapy" means decrease, suppress, attenuate, diminish, arrest, or stabilize the development or progression of a disease (e.g., a disease or disorder delineated herein), lessen the severity of the disease or improve the symptoms associated with the disease. Additionally, it includes prophylactic use. The treatment or therapy may be on humans or animals.

The compound will be formulated to contain deuterated compound in an amount such that, depending on the route of administration and prescribed frequency, the required daily or weekly dosage of deuterated compound is provided. The composition will be formulated to allow for daily administration or so it can be incorporated in a treatment regime. The compounds may be used on their own or formulated in combination with other therapeutic compounds for the treatment of motor neurone disease.

"Motor Neurone Disease" in the context used herein is understood to be a disorder selected from Amyotrophic Lateral Sclerosis (ALS), Primary Lateral Sclerosis (PLS), Progressive Lateral Sclerosis (PLS), Progressive Bulbar Palsy (PBP), pseudobulbar palsy, Spinal Muscular Atrophy (SMA), spinobulbar muscular atrophy, Charcot-Marie-Tooth disease and other degenerative disorders of upper and/or lower spinal motor neurones irrespective of whether they are classified as "motor neurone disease" in commonly used disease classification systems such as Medical Subject Headings (MeSH) or the International Statistical Classification of Diseases and Related Health Problems (ICD-10). It is also understood that motor neurone diseases may be known by alternative names and/or is subject to different classification in territories other than the United Kingdom (for example Lou Gehrig's disease in the United States).

When they are used, the compounds are included in a "pharmaceutically acceptable" carrier that means a component that is, within the scope of sound medical judgment, suitable for use in contact with the tissues of humans and other mammals without undue toxicity, irritation, allergic response and the like, and are commensurate with a reasonable benefit/risk ratio.

Such carriers include "pharmaceutically acceptable salts" means any non-toxic salt that, upon administration to a recipient, is capable of providing, either directly or indirectly, a compound of this invention. A "pharmaceutically acceptable counter-ion" is an ionic portion of a salt that is not toxic when released from the salt upon administration to a recipient. Acids commonly employed to form pharmaceutically acceptable salts include inorganic acids such as hydrogen bisulfide, hydrochloric acid, hydrobromic acid, hydroiodic acid, sulfuric acid and phosphoric acid, as well as organic acids such as para-toluenesulfonic acid, salicylic acid, tartaric acid, bitartaric acid, ascorbic acid, maleic acid, besylic acid, fumaric acid, gluconic acid, glucuronic acid, formic acid, glutamic acid, methanesulfonic acid, ethanesulfonic acid, benzenesulfonic acid, lactic acid, oxalic acid, para-bromophenylsulfonic acid, carbonic acid, succinic acid, citric acid, benzoic acid and acetic acid, as well as related inorganic and organic acids. A salt of a compound of this invention is formed between an acid and a basic group of the compound, such as an amino functional group, or a base and an acidic group of the compound, such as a carboxyl functional group. Pharmaceutically acceptable salts thus include but are not limited to sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, terephthalate, sulfonate, xylene sulfonate, phenylacetate, phenylpropionate, phenylbutyrate, citrate, lactate, [beta]-hydroxybutyrate, glycolate, maleate, tartrate, methanesulfonate, propanesulfonate, naphthalene-1-sulfonate, naphthalene-2-sulfonate, mandelate and other salts. Pharmaceutically acceptable acid addition salts include those formed with mineral acids such as hydrochloric acid and hydrobromic acid, and especially those formed with organic acids such as maleic acid. The pharmaceutically acceptable salt may also be a salt of a compound of the present invention having an acidic functional group, such as a carboxylic acid functional group, and a base. Exemplary bases include, but are not limited to, hydroxide of alkali metals including sodium, potassium, and lithium; hydroxides of alkaline earth metals such as calcium and magnesium; hydroxides of other metals, such as aluminium and zinc; ammonia, organic amines such as unsubstituted or hydroxyl-substituted mono-, di-, or trialkylamines, dicyclohexylamine; tributyl amine; pyridine; N-methyl, N-ethylamine; diethylamine; triethylamine; mono-, bis-, or tris-(2-OH-(Ci-C6)-alkylamine), such as N,N-dimethyl-N-(2-hydroxyethyl)amine or tri-(2-hydroxyethyl)amine; N-methyl-D- glucamine; morpholine; thiomorpholine; piperidine; pyrrolidine; and amino acids such as arginine, lysine, and the like.

The invention also provides pharmaceutical compositions comprising an effective amount of a compound of Formula (I), (II), (III), (IV), (V), (VI), (VII), (VIII) or (IX) (e.g., including any of the formulae herein), or a pharmaceutically acceptable salt of said compound; and a pharmaceutically acceptable carrier. The carrier(s) are "acceptable" in the sense of being compatible with the other ingredients of the formulation and, in the case of a pharmaceutically acceptable carrier, not deleterious to the recipient thereof in an amount used in the medicament.

Pharmaceutically acceptable carriers, adjuvants and vehicles that may be used in the pharmaceutical compositions of this invention include, but are not limited to, ion exchangers, alumina, aluminium stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene -block polymers, polyethylene glycol and wool fat. It is also possible to, enhance the bioavailability if the compound using an amorphous form of the compound optionally formulated with a poloxamer, such as LUTPvOL(TM) and PLURONIC(TM) (BASF Corporation), or block copolymers of ethylene oxide and propylene oxide.

The compound of the present invention may exist in a number of different crystalline polymorphs and the polymorph(s) with optimal pharmaceutical properties can be selected so all crystalline polymorphs of the compound of the present invention are incorporated herein.

The pharmaceutical compositions of the invention include those suitable for oral, rectal, nasal, topical (including buccal and sublingual), vaginal or parenteral (including subcutaneous, intramuscular, intravenous and intradermal) intrathecal or other intraspinal administration. In certain embodiments, the compound of the formulae herein is administered transdermally (e.g., using a transdermal patch or iontophoretic techniques). Other formulations may conveniently be presented in unit dosage form, e.g., tablets, sustained release capsules, and in liposomes. The molecule to be administered is brought into association with ingredients such as the carrier which may be a liquid carrier, liposomes or finely divided solid carriers, or both, and then, if necessary, shaping the product.

Compositions of the present invention suitable for oral administration may be presented as discrete units such as capsules, sachets, or tablets each containing a predetermined amount of the active ingredient; a powder or granules; a solution or a suspension in an aqueous liquid or a nonaqueous liquid; an oil-in-water liquid emulsion; a water-in-oil liquid emulsion; packed in liposomes; or as a bolus, etc. Soft gelatin capsules can be useful for containing such suspensions, which may beneficially increase the rate of compound absorption. In the case of tablets for oral use, carriers that are commonly used include lactose and corn starch. Lubricating agents, such as magnesium stearate, are also typically added. For oral administration in a capsule form, useful diluents include lactose and dried cornstarch. When aqueous suspensions are administered orally, the active ingredient is combined with emulsifying and suspending agents. If desired, certain sweetening and/or flavoring and/or colouring agents may be added, for example if the composition is included in a lozenges a flavouring which is usually sucrose and acacia or tragacanth is used and for pastilles the active ingredient is included with an inert basis such as gelatin and glycerin, or sucrose and acacia.

Compositions suitable for parenteral administration include aqueous and nonaqueous sterile injection solutions which may contain anti-oxidants, buffers, bacteriostats and solutes which render the formulation isotonic with the blood of the intended recipient; and aqueous and nonaqueous sterile suspensions which may include suspending agents and thickening agents. The formulations may be presented in unit- dose or multi-dose containers, for example, sealed ampules and vials, and may be stored in a freeze dried (lyophilized) condition requiring only the addition of the sterile liquid carrier, for example water for injections, immediately prior to use. Extemporaneous injection solutions and suspensions may be prepared from sterile powders, granules and tablets. Such injection solutions may be in the form, for example, of a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally-acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long- chain alcohol diluent or dispersant.

The pharmaceutical compositions of this invention may be administered in the form of suppositories for rectal administration. These compositions can be prepared by mixing a compound of this invention with a suitable non-irritating excipient which is solid at room temperature but liquid at the rectal temperature and therefore will melt in the rectum to release the active components. Such materials include, but are not limited to, cocoa butter, beeswax and polyethylene glycols.
The pharmaceutical compositions of this invention may be administered by nasal aerosol or inhalation. Such compositions are prepared according to techniques well- known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

Topical administration of the pharmaceutical compositions of this invention is especially useful when the desired treatment involves areas or organs readily accessible by topical application. For topical application topically to the skin, the pharmaceutical composition should be formulated with a suitable ointment containing the active components suspended or dissolved in a carrier. Carriers for topical administration of the compounds of this invention include, but are not limited to, mineral oil, liquid petroleum, white petroleum, propylene glycol, polyoxyethylene polyoxypropylene compound, emulsifying wax, and water. Alternatively, the pharmaceutical composition can be formulated with a suitable lotion or cream containing the active compound suspended or dissolved in a carrier. Suitable carriers include, but are not limited to, mineral oil, sorbitan monostearate, polysorbate 60, cetyl esters wax, cetearyl alcohol, 2- octyldodecanol, benzyl alcohol, and water. The pharmaceutical compositions of this invention may also be topically applied to the lower intestinal tract by rectal suppository formulation or in a suitable enema formulation. Topically-transdermal patches and iontophoretic administration are also included in this invention.

Application of the subject therapeutics may be local, so as to be administered at the site of interest. Various techniques can be used for providing the subject compositions at the site of interest, such as injection, use of catheters, trocars, projectiles, pluronic gel, stents, sustained drug release polymers or other device which provides for internal access. Accordingly, the compounds of this invention may be incorporated into compositions for coating an implantable medical device, such as prostheses, artificial valves, vascular grafts, stents, or catheters. Suitable coatings are typically biocompatible polymeric materials such as a hydrogel polymer, polymethyldisiloxane, polycaprolactone, polyethylene glycol, polylactic acid, ethylene vinyl acetate, and mixtures thereof. The coatings may optionally be further covered by a suitable topcoat of fluorosilicone, polysaccharides, polyethylene glycol, phospholipids or combinations thereof to impart controlled release characteristics in the composition.

The compound of the present invention is administered in an effective amount which means that when administered in a proper dosing regimen, the amount is sufficient to treat the target disorder and this amount can vary according to the height and weight of the subject. Effective doses of the compounds of the present invention will also vary, as recognized by those skilled in the art, depending on the disease(s) treated, the severity of the disease, the route of administration, the sex, age and general health condition of the subject, excipient usage, the possibility of co- usage with other therapeutic treatments such as use of other agents and the judgment of the treating physician.

It is also possible to co-administer the compound of the invention with one or more second therapeutic agents or treatments. The term "co-administered" as used herein means that the second therapeutic agent may be administered together with a compound of this invention as part of a single dosage form (such as a composition of this invention comprising a compound of the invention and an second therapeutic agent as described above) or as separate, multiple dosage forms. Alternatively, the additional agent may be administered prior to, consecutively with, or following the administration of a compound of this invention. It is possible that where a second therapeutic agent is administered to a subject, the effective amount of the compound of the invention or the second therapeutic agent that is required is less than its effective amount than when the agent or compound are administered on their own so by being able to give less drugs to the individual undesired side effects associated with high doses of either agent may be minimized. Other potential advantages include improved dosing regimens and/or reduced drug cost.

The present invention overcomes problems of the prior art as there is a reduction in metabolism of the compound compared with that of its non-deuterated parent compound. This has the surprising effect that the specific deuterated compounds described are more neuroprotective compared with their non-deuterated parent compounds and also give rise to lower levels of neurotoxic metabolites so making the compound particularly attractive for administering to patients that have a long term illness.

### Examples

### Synthesis

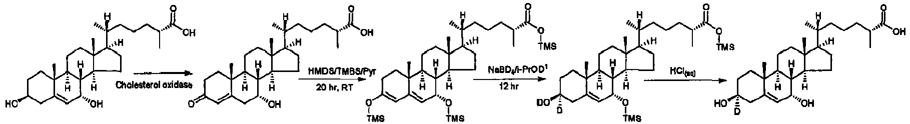

### Scheme 1. Partial synthesis of [3α-²H]3[β,7α-dihydroxycholest-5-en-26-oic acid. HMDS, hexamethyldisilazane; TMBS, trimethylbromosilane; Pyr, pyridine; i-PrOD, [O²H]isopropanol.

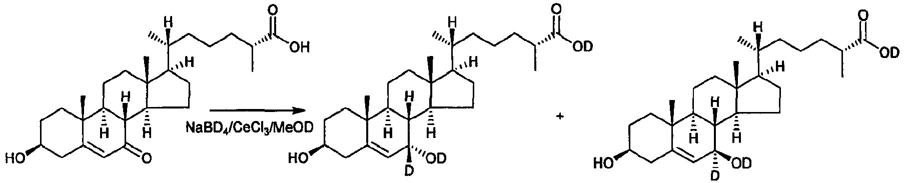

### Scheme 2. Luche reduction of the 7-oxo group of 3β-hydroxy-7-oxocholest-5-en-26-oic acid to [7β-²H]3β,7α-dihydroxycholest-5-en-26-oic acid and [7α-²H]3β,7β-dihydroxycholest-5-en-26-oic acid.

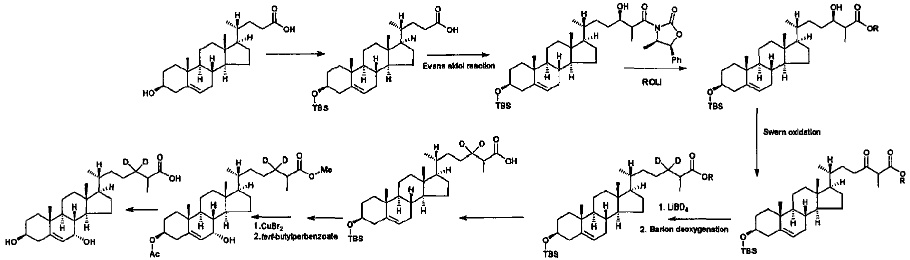

### Scheme 3. Partial synthesis of [24,24-²H₂]3β,7α-dihydroxycholest-5-enoic acid.

### Evaluation of Metabolic Stability

The metabolic stability of the deuterated compounds can be compared to that of unlabelled compounds by in *vitro* incubation (i) with the enzyme HSD3B7 to assess the propensity for oxidation at C-3, (ii) with the enzyme HSD11B1, a putative 7-hydroxy epimerase, and (iii) with peroxisome preparation to asses the propensity for side-chain shortening; followed by mass spectrometry analysis to determine the rate of loss of substrate and the rate of formation products. Similar experiments can be performed with primary cultures of mouse and human hepatocytes, and of mouse neurons, glial and Schwann cells.

To asses in *vivo* metabolism in brain, mice can be injected with both deuterated compounds and also with analogues ¹³C labelled compounds. The presence of both substrates and products can be measured in cerebrospinal fluid and brain tissue, and in both the carotid artery and jugular vein, by mass spectrometry providing a measure of the relative rate of metabolism.

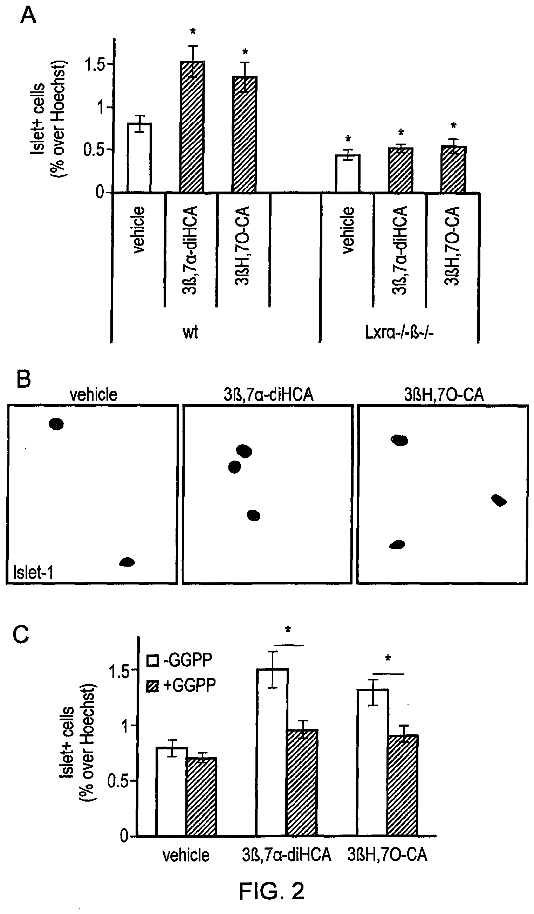

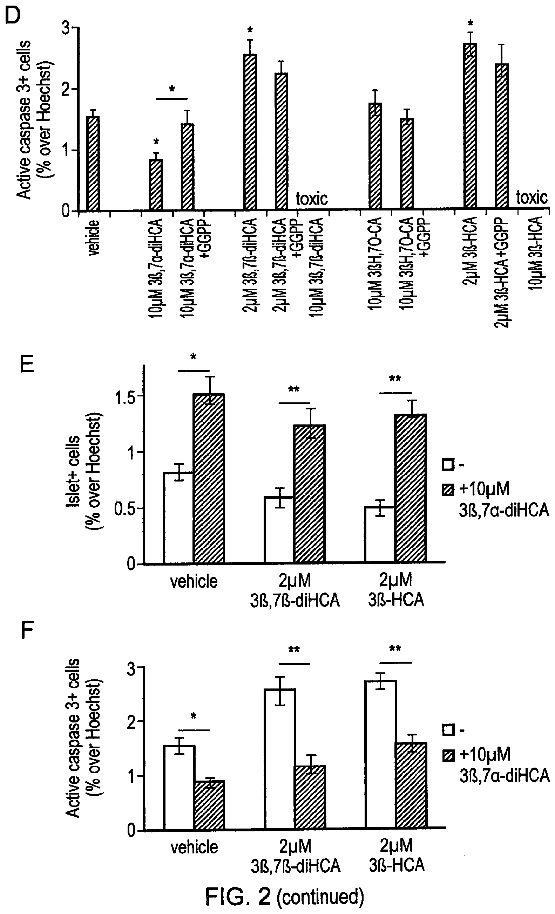

As shown in **Figure 2****.** (A) Quantification of Islet1 + cells in mouse E11.5 brain primary cultures from wild type (wt) or Lxrα-/-β-/- embryos treated with 3β,7α-dihydroxycholest-5-en-26-oic acid (3β,7α-diHCA) or 3β-hydroxy-7-oxocholest-5-en-26-oic acid (3βH,7O-CA). Data are means ± SE (n = 3), *, p < 0.05 compared to vehicle wt group. (B) Representative images of Islet-1 stained cell nuclei, and (C) the effect of the Lxr antagonist geranylgeranyl pyrophosphate (GGPP, 10 µM) on the treatments in the wt group. (D) Quantification of active caspase 3+ cells in mouse E11.5 brain primary cultures (wt group) treated with 2 or 10 µM of the acids as indicated, with or without 10 µM GGPP. Data are means ± SE (n = 3), *, p < 0.05 compared to vehicle treatment or compared to '-GGPP' group as indicated. (E,F) 10 µM 3β,7α-diHCA rescues the toxic effect of 2 µM 3β,7β-diHCA or 3β-HCA on Islet1+ cells (E) and reduces neuronal cell death induced by these acids as indicated by the number of active caspase 3+ cells (F) in mouse E11.5 brain primary cultures (wt group). Data represent mean ± SE (n = 3), *, p <0.05; **, p < 0.01 as indicated.

It should be noted that the above mentioned embodiment illustrates rather than limits the invention. It is to be noted that the invention covers not only individual embodiments described but also combinations of those embodiments.

## Claims

1. A compound of general formula (I) 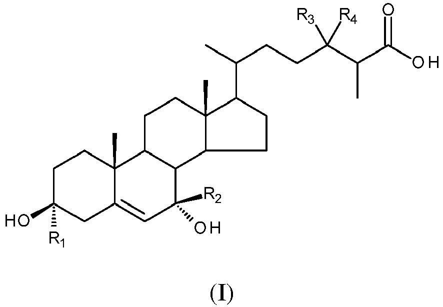 or a pharmaceutically acceptable salt thereof, wherein R₁ - R₄ are each independently selected from H and deuterium; and at least one of R₁ - R₄ is deuterium.

2. A compound according to claim 1 wherein R₁ is deuterium and R₂, R₃ and R₄ are hydrogen.

3. A compound according to claim 1 wherein R₂ is deuterium and R₁, R₃, R₄ are hydrogen.

4. A compound according to claim 1 wherein R₁ and R₂ are deuterium and R₃ and R₄ are hydrogen.

5. A compound according to Claim 1, wherein R₃ and R₄ are deuterium and R₁ and R₂ are hydrogen.

6. A compound according to Claim 1, where R₁, R₂, R₃ and R₄ are deuterium.

7. A compound of any preceding claim which is a deuterated cholestenoic acid or a pharmaceutically acceptable salt thereof.

8. A compound of any preceding claim wherein the cholestenoic acid is either a) deuterated 3β,7α-dihydroxycholest-5-en-26-oic acid (3β,7α-diHCA) or b) 3α,7β-dideutero, 3β,7α-dihyroxycholest-5-en-oic acid or a pharmaceutically acceptable salt thereof.

9. A compound according to any preceding claim which is an inhibitor of an epimerase that converts 3β,7α-dihydroxycholest-5-en-26-oic acid (3β,7α-diHCA) to 3β, 7β-dihydroxycholest-5-en-26-oic acid (3β,7β-diHCA).

10. A compound according to any preceding claim for the treatment of neurodegenerative conditions.

11. A pharmaceutical or veterinary composition comprising a compound as claimed in any of claims 1 to 9 in conjunction or association with a pharmaceutically or veterinarily acceptable carrier or vehicle.

12. A pharmaceutical or veterinary composition according to claim 11 which is suitable for oral, rectal, nasal, bronchial, topical, transdermal or parenteral administration.

13. A compound as claimed in any one of claims 1 to 9 or a composition as claimed in claim 11 or 12 for use in a method of treatment or prevention of neurodegenerative conditions, which method comprises modifying the amount of specific cholestenoic acids in an individual by administering to an individual a compound as claimed in any one of claims 1 to 9 or a composition as claimed in claim 11 or 12.

14. A compound or composition for use according to claim 13, for the treatment of a neurodegenerative condition and in particular where the neurodegenerative condition is a systemic atrophy, a muscular atrophy, an atrophy of the Central Nervous system or a combination thereof and in particular amyotrophic lateral sclerosis (ALS), primary lateral sclerosis (PLS), progressive muscular atrophy (PMA), progressive bulbar palsy (PBP), Pseudobulbar palsy (BP), spinal muscular atrophy (SMA) hereditary spastic paresis (HSP) or cerebrotendinous xanthomatosis (CTX).

## Patentansprüche

1. Verbindung der allgemeinen Formel (I) 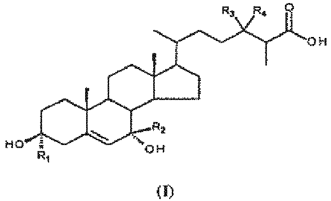 oder ein pharmazeutisch verträgliches Salz davon, wobei R₁ - R₄ jeweils unabhängig voneinander ausgewählt sind unter H und Deuterium und mindestens einer von R₁ - R₄ für Deuterium steht.

2. Verbindung nach Anspruch 1, wobei R₁ für Deuterium und R₂, R₃ und R₄ für Wasserstoff stehen.

3. Verbindung nach Anspruch 1, wobei R₂ für Deuterium und R₁, R₃ und R₄ für Wasserstoff stehen.

4. Verbindung nach Anspruch 1, wobei R₁ und R₂ für Deuterium und R₃ und R₄ für Wasserstoff stehen.

5. Verbindung nach Anspruch 1, wobei R₃ und R₄ für Deuterium und R₁ und R₂ für Wasserstoff stehen.

6. Verbindung nach Anspruch 1, wobei R₁, R₂, R₃ und R₄ für Deuterium stehen.

7. Verbindung nach einem der vorhergehenden Ansprüche, die eine deuterierte Cholestensäure oder ein pharmazeutisch verträgliches Salz davon ist.

8. Verbindung nach einem der vorhergehenden Ansprüche, wobei die Cholestensäure entweder a) deuterierte 3β,7α-dihydroxycholest-5-en-26-Säure (3β,7α-diHCA) oder b) 3α,7β-dideutero,3β,7α-dihydroxycholest-5-en-Säure oder ein pharmazeutisch verträgliches Salz von diesen ist.

9. Verbindung nach einem der vorhergehenden Ansprüche, die ein Inhibitor einer Epimerase ist, die 3β,7α-dihydroxycholest-5-en-26-Säure (3β,7α-diHCA) in 3β,7β-dihydroxycholest-5-en-26-Säure (3β,7β-diHCA) umwandelt.

10. Verbindung nach einem der vorhergehenden Ansprüche zu Behandlung von neurodegenerativen Erkrankungen.

11. Pharmazeutische oder veterinäre Zusammensetzung, umfassend eine Verbindung nach einem der Ansprüche 1 bis 9 in Verbindung oder in Assoziation mit einem pharmazeutisch oder veterinär verträglichen Träger oder Vehikel.

12. Pharmazeutische oder veterinäre Zusammensetzung nach Anspruch 11, die für eine orale, rektale, nasale, bronchiale, topische, transdermale oder parenterale Anwendung geeignet ist.

13. Verbindung nach einem der Ansprüche 1 bis 9 oder Zusammensetzung nach Anspruch 11 oder 12 zur Verwendung in einem Verfahren zur Behandlung oder Vorbeugung neurodegenerativer Erkrankungen, wobei das Verfahren das Modifizieren der Menge der spezifischen Cholestensäure in einem Individuum durch Verabreichung an das Individuum einer Verbindung nach einem der Ansprüche 1 bis 9 oder einer Zusammensetzung nach Anspruch 11 oder 12 umfasst.

14. Verbindung oder Zusammensetzung zur Verwendung nach Anspruch 13 zur Behandlung einer neurodegenerativen Erkrankung und insbesondere wobei die neurodegenerative Erkrankung eine systemische Atrophie, eine muskuläre Atrophie, eine Atrophie des zentralen Nervensystems oder eine Kombination davon ist und insbesondere amyotrophe Lateralsklerose (ALS), primäre Lateralsklerose (PLS), progressive Muskelatrophie (PMA), progressive Bulbärparalyse (PBP), Pseudobulbärparalyse (BP), spinale Muskelatrophie (SMA), heriditäre spastische Paraplegie (HSP) oder zerebrotendinöse Xanthomatose (CTX).

## Revendications

1. Composé de formule générale (I) 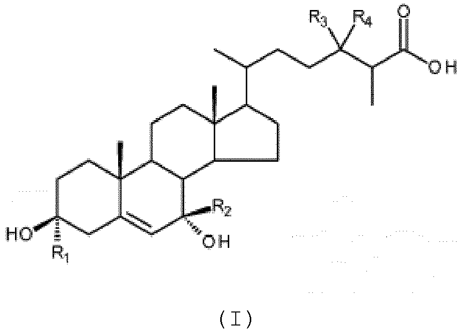 ou un sel pharmaceutiquement acceptable de celui-ci, dans laquelle R₁ à R₄ sont chacun indépendamment choisis parmi H et un atome de deutérium ; et au moins un de R₁ à R₄ est un atome de deutérium.

2. Composé selon la revendication 1, dans lequel R₁ est un atome de deutérium et R₂, R₃ et R₄ sont un atome d'hydrogène.

3. Composé selon la revendication 1, dans lequel R₂ est un atome de deutérium et R₁, R₃ et R₄ sont un atome d'hydrogène.

4. Composé selon la revendication 1, dans lequel R₁ et R₂ sont un atome de deutérium et R₃ et R₄ sont un atome d'hydrogène.

5. Composé selon la revendication 1, dans lequel R₃ et R₄ sont un atome de deutérium et R₁ et R₂ sont un atome d'hydrogène.

6. Composé selon la revendication 1, dans lequel R₁, R₂, R₃ et R₄ sont un atome de deutérium.

7. Composé selon l'une quelconque des revendications précédentes, qui est un acide cholesténoïque deutérié ou un sel pharmaceutiquement acceptable de celui-ci.

8. Composé selon l'une quelconque des revendications précédentes, dans lequel l'acide cholesténoïque est a) l'acide 3β,7α-dihydroxycholest-5-én-26-oïque (3β,7α-diHCA) deutérié ou b) l'acide 3α,7β-deutério-3β,7α-dihydroxycholest-5-énoïque ou un sel pharmaceutiquement acceptable de ceux-ci.

9. Composé selon l'une quelconque des revendications précédentes, qui est un inhibiteur d'une épimérase qui convertit l'acide 3β,7α-dihydroxycholest-5-én-26-oïque (3β,7α-diHCA) en acide 3β,7β-dihydroxycholest-5-én-26-oïque (3β,7β-diHCA).

10. Composé selon l'une quelconque des revendications précédentes pour le traitement de pathologies neurodégénératives.

11. Composition pharmaceutique ou vétérinaire comprenant un composé selon l'une quelconque des revendications 1 à 9 conjointement ou en association avec un excipient ou un véhicule acceptable d'un point de vue pharmaceutique ou vétérinaire.

12. Composition pharmaceutique ou vétérinaire selon la revendication 11, qui est appropriée pour une administration orale, rectale, nasale, bronchique, topique, transdermique ou parentérale.

13. Composé selon l'une quelconque des revendications 1 à 9 ou composition selon la revendication 11 ou 12 pour une utilisation dans une méthode de traitement ou de prévention de pathologies neurodégénératives, laquelle méthode comprend la modification de la quantité d'acides cholesténoïques spécifiques chez un individu par administration par un individu d'un composé selon l'une quelconque des revendications 1 à 9 ou d'une composition selon la revendication 11 ou 12.

14. Composé ou composition pour une utilisation selon la revendication 13, pour le traitement d'une pathologie neurodégénérative et en particulier lorsque la pathologie neurodégénérative est une atrophie systémique, une atrophie musculaire, une atrophie du système nerveux central ou une combinaison de celles-ci et en particulier la sclérose latérale amyotrophique (SLA), la sclérose latérale primaire (SLP), l'atrophie musculaire progressive (AMP), la paralysie bulbaire progressive (PBP), la paralysie pseudobulbaire (BP), l'atrophie musculaire spinale (AMS), la parésie spastique héréditaire (PSH) ou la xanthomatose cérébrodentineuse (CTX).
